# EUROPEAN PATENT SPECIFICATION

(11) **EP 4 533 057 B1**
(45) Date of publication and mention of the grant of the patent: **02.12.2026**
(21) Application number: 23734804.0
(22) Date of filing: 30.05.2023
(51) Int. Cl.: G01N 1/30, G01N 33/50, C12Q 1/68, C12Q 1/6804, C12Q 1/6841

(54) **METHODS AND SYSTEMS FOR REMOVING A HISTOLOGICAL STAIN FROM A SAMPLE**
METHODEN UND SYSTEME ZUR ENTFERNUNG EINER HISTOLOGISCHEN FÄRBUNG AUS EINER PROBE
MÉTHODES ET SYSTÈMES POUR ÉLIMINER UNE COLORATION HISTOLOGIQUE D'UN ÉCHANTILLON

(30) Priority: 31.05.2022 US 202263347150 P; 22.12.2022 US 202263434578 P
(43) Date of publication of application: 09.04.2025
(73) Proprietor: Ultivue, Inc., Cambridge, Massachusetts 02138 (US)
(72) Inventor: HWANG, Kevin, Cambridge, Massachusetts 02138 (US); VEZEAU, Grace Elizabeth, Cambridge, Massachusetts 02138 (US); KRZYMANSKA-OLEJNIK, Edyta, Cambridge, Massachusetts 02138 (US)
(74) Representative: Mewburn Ellis LLP
(86) International application number: PCT/US2023/023811
(87) International publication number: WO 2023/235284

(56) References cited:
- WO-A1-2013/088693
- US-A1- 2013 236 984
- US-A1- 2021 238 675
- HENRICHS D W ET AL: "PCR Amplification of Microsatellites from Single Cells of Karenia brevis Preserved in Lugol's Iodine Solution", MARINE BIOTECHNOLOGY, SPRINGER-VERLAG, NE, vol. 10, no. 2, 7 October 2007 (2007-10-07), pages 122 - 127, XP019566893, ISSN: 1436-2236

## Description

### CROSS-REFERENCE TO RELATED APPLICATIONS

This application claims the benefit of priority of US Provisional Application No. 63/347,150, filed May 31, 2022, and US Provisional Application No. 63/434,578, filed December 22, 2022.

### DESCRIPTION

### FIELD

The present invention relates to a method and a kit for removing a histological stain from a biological sample. The methods are applicable, for example, to removing hematoxylin from a tissue sample.

### INTRODUCTION

Hematoxylin and eosin (H&E) staining is well established in the field of histopathology. H&E staining is used for the evaluation of cellular morphology and is a primary tool used by pathologists for diagnosing cancer. To obtain further information about pathology, other analysis methods such as immunohistochemistry (IHC) and in situ hybridization are commonly carried out to probe protein or nucleic acid targets. This analysis is often performed on adjacent thin sections cut from the same tissue sample. However, such adjacent sections are different from each other at the single cell level, and tissue samples are sometimes in short supply (e.g., tissues from needle-stick biopsies), and as a result, technologies for using a single tissue section for multiple analyses are of interest.

One of the major problems with using an H&E-stained sample for subsequent analysis is the interference from H&E stains in detecting subsequent labels, e.g., chromogenic or fluorescence labels, used for detection in methods such as IHC and in situ hybridization. In some instances, attempts have been made to perform fluorescence IHC using near-infrared dyes having emissions far removed from the H&E fluorescence emission; however, the available spectral space for such dyes is very limited. Most commercially available fluorescent probes and labeled antibodies are labeled with fluorophores having emissions overlapping with H&E emission.

Methods for H&E stain removal have been reported to enable subsequent IHC staining on an H&E-de-stained tissue section. However, such attempts have been unsuccessful for multi-analyte chromogenic IHC and fluorescence IHC because, for example, hematoxylin and eosin may not be fully removed using the existing methods. Even small amounts of residual hematoxylin staining can be demonstrated to broadly interfere with detection of fluorescence signals over background. In some instances, the workflow for hematoxylin removal using strong acid has been suggested; however, the time necessary for the removal may take an hour to hours and such a lengthy treatment with the strong acid may degrade the sample, making it no longer suitable for subsequent analysis using another method.

Accordingly, there remains a need for a method to remove bound hematoxylin from biological samples efficiently, which allows the same sample to be used for subsequent analysis, such as IHC or in situ hybridization and other chromogenic and fluorescence-based methods for detecting biological molecules such as nucleic acids and proteins.

### SUMMARY

The present invention relates to a method and a kit for removing a histological stain (e.g., hematoxylin) from a stained biological sample, such as a cell- or tissue-containing sample. In certain embodiments, the removal of hematoxylin stain is accomplished by treatment with a strong acid followed by treatment with a reducing agent. In some embodiments, this process is accomplished in less than one hour.

The present disclosure also provides methods for detecting multiple targets in a sample. The method may include optionally detecting hematoxylin staining of the sample; removing the hematoxylin staining from the stained sample; and detecting additional features or targets in the sample. Additional features may include any morphological feature, such as connective tissue, fat, amyloid, myelin, etc. that may be detected using an analysis method. Additional targets may include any proteins, nucleic acids, or any other molecular targets that may be detected using an analysis method.

### BRIEF DESCRIPTION OF THE DRAWINGS

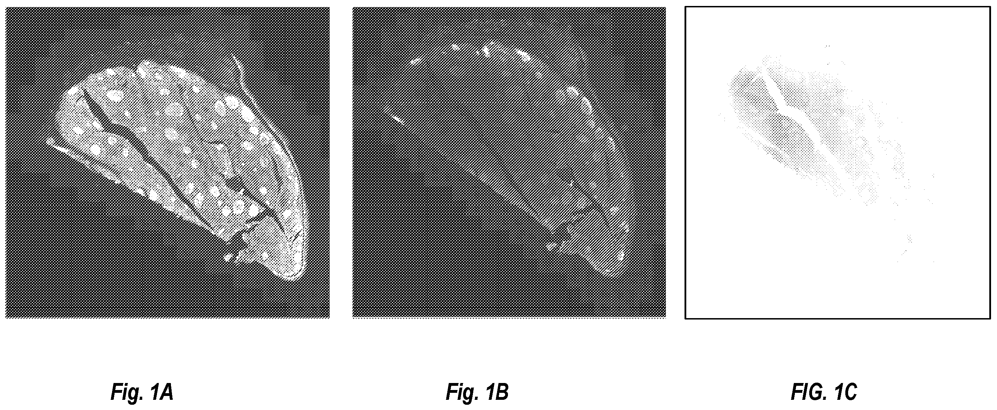

Figures 1A-1C show images of tissue stained using multiplexed IHC. Figure 1A shows a fluorescence image of a positive control (where no H&E staining was performed prior to IHC); Figure 1B shows a fluorescence image of a negative control (where H&E staining was performed prior to IHC but no hematoxylin removal was performed); and Figure 1C shows a brightfield image of the negative control, according to Example 1.

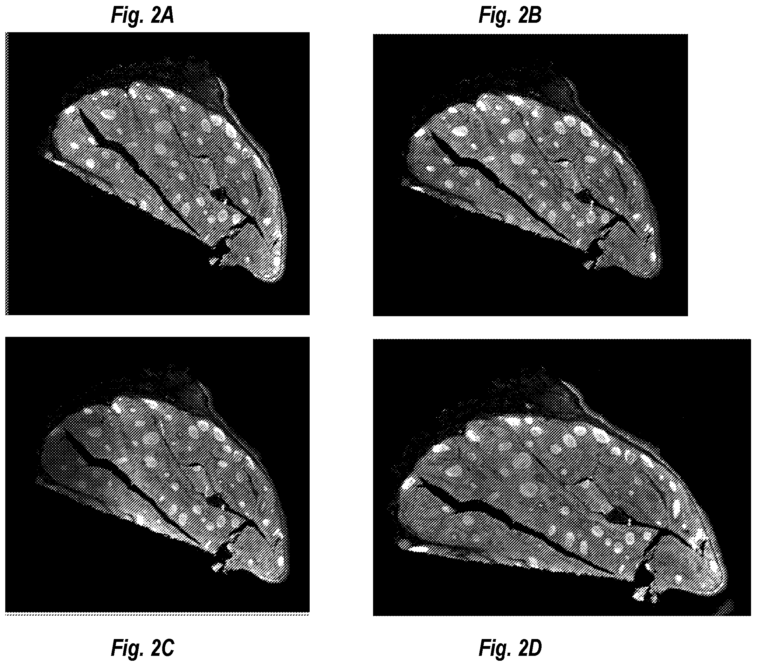

Figures 2A-2D show fluorescent images of tissues stained with IHC where no H&E staining was performed prior to IHC staining and no treatment (Fig. 2A; slide #01); the reducing agent treatment (Fig. 2B; slide #03); the acidic agent treatment (Fig. 2D; slide #07); or both treatments (Fig. 2C; slide #05) were performed according to Example 1.

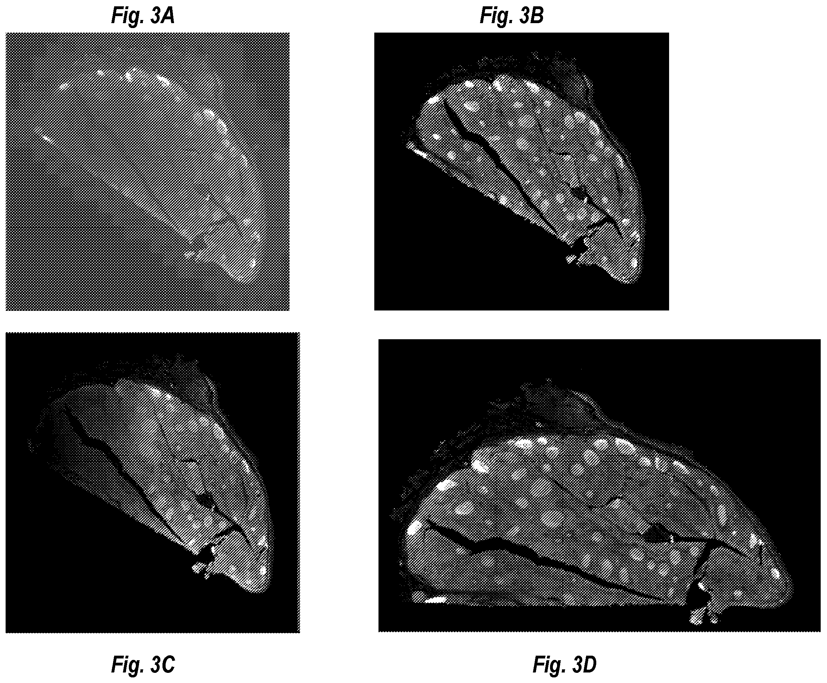

Figures 3A-3D show fluorescent images of tissues stained with IHC where H&E staining was performed followed by IHC staining but no treatment (Fig. 3A; slide #02), the reducing agent treatment (Fig. 3B; slide #04), the acidic agent treatment (Fig. 3D; slide #08), or both treatments (Fig. 3C; slide #06) were performed according to Example 1.

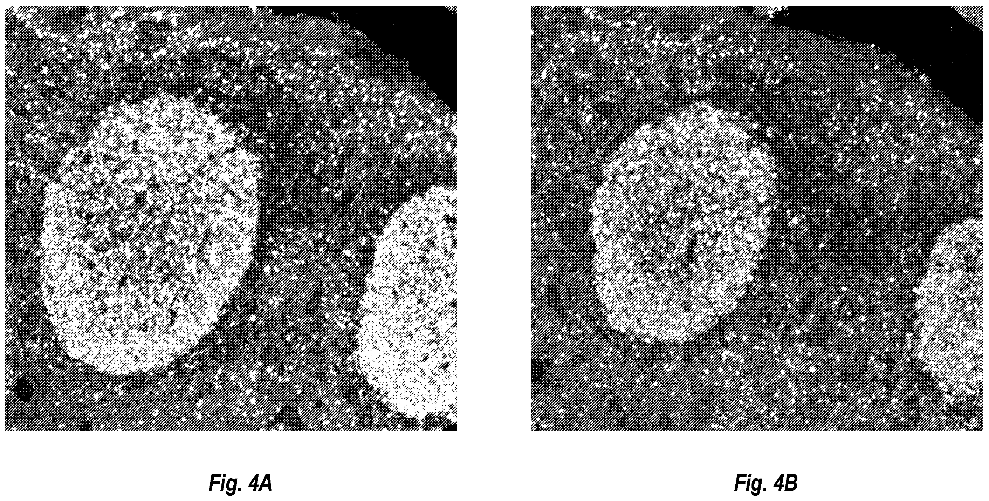

Figures 4A-4B show fluorescence images of tissue subjected to immunostaining with the four different probes according to Example 1 under two conditions: (Fig. 4A) no H&E, no treatment (slide #01; positive control, in which the tissue slide was not H&E-stained); (Fig. 4B) removal of H&E staining from a previously H&E-stained slide according to the present disclosure (slide #06).

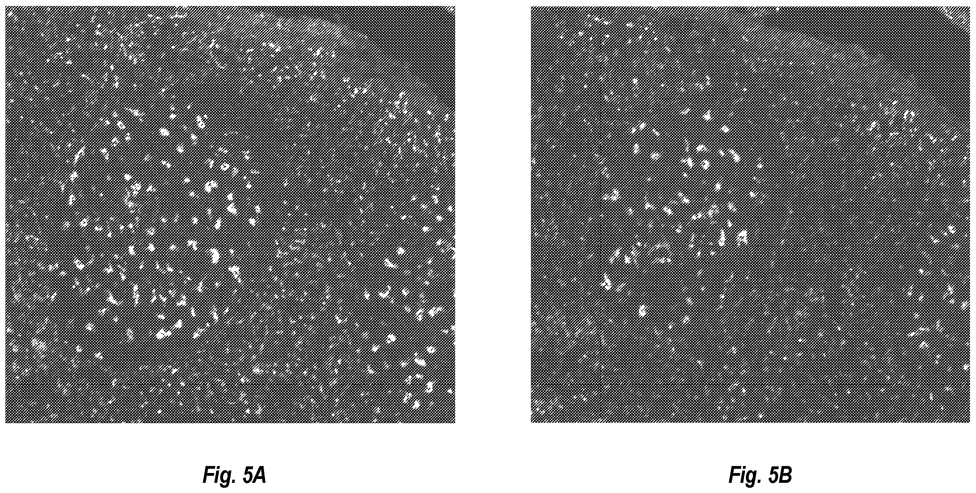

Figures 5A-5B show fluorescence images of single-channel detection in Cy7 fluorescent channel with dye-labeled probe for CD68 from the tissue treated according to Example 1: (Fig. 5A) no H&E, no treatment (slide #01; positive control) and (Fig. 5B) H&E, acidic agent and reducing agent (slide #06).

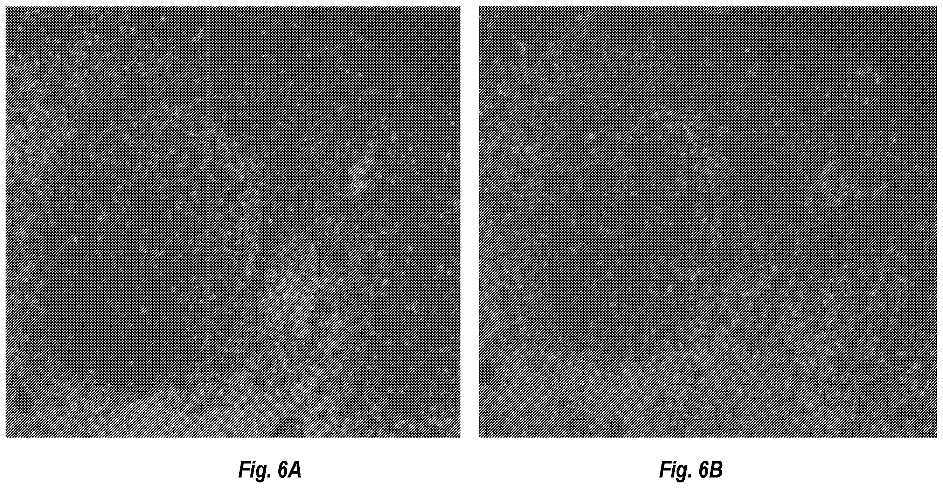

Figures 6A-6B show fluorescence images of single-channel detection of Cy5 fluorescent channel with dye-labeled probe for CD3 from the tissue treated according to Example 1: (Fig. 6A) no H&E, no treatment (slide #01; positive control) and (Fig. 6B) H&E, acidic agent and reducing agent.

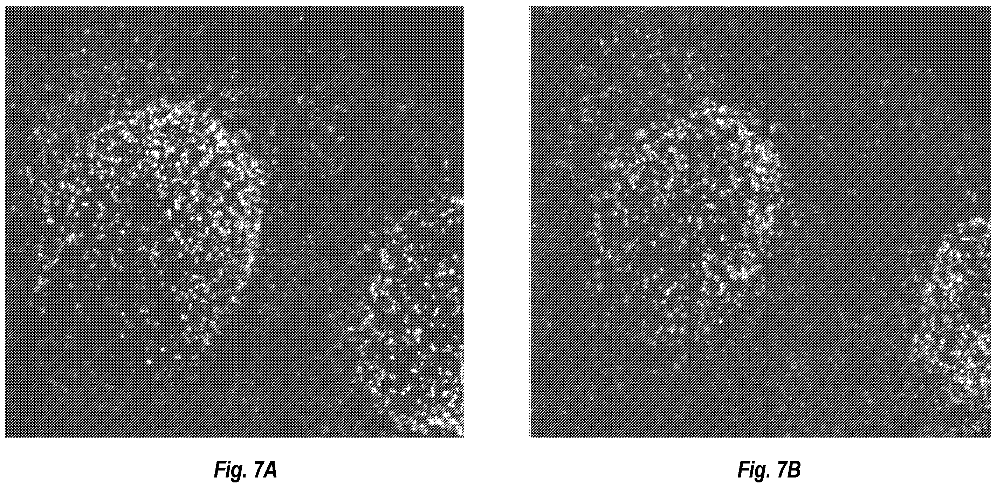

Figures 7A-7B show fluorescence images of single-channel detection of TRITC fluorescent channel with dye-labeled probe for PD1 from the tissue treated according to Example 1: (Fig. 7A) no H&E, no treatment (slide #01; positive control) and (Fig. 7B) H&E, acidic agent and reducing agent.

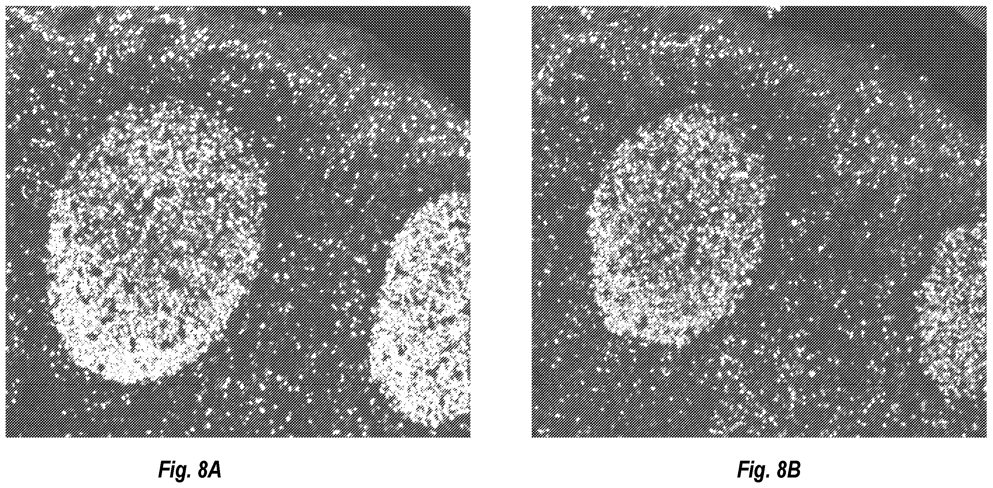

Figures 8A-8B show fluorescence images of single-channel detection of FITC fluorescent channel using dye-labeled probe for Ki67 from the tissue treated according to Example 1: (Fig. 8A) no H&E, no treatment (slide #01; positive control) and (Fig. 8B) H&E, acidic agent and reducing agent.

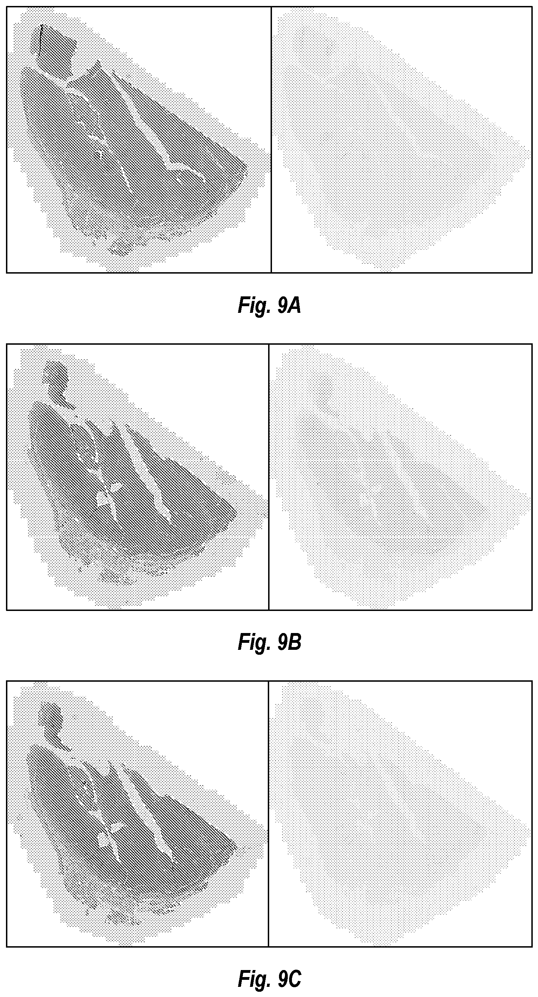

Figures 9A-9F show bright field images of tissues de-stained using different conditions of reducing agent according to Example 2. The left panels of Figs. 9A-9F show the bright field images prior to the treatment and the right panels of Figs. 9A-9F show the brightfield images after the removal treatment.

### DESCRIPTION OF THE EMBODIMENTS

### I. Exemplary Methods

In some embodiments, a method of removing a histological stain from a histologically stained sample is provided.

In certain embodiments, the removal of the histological stain is accomplished by: (a) contacting the sample with an acidic agent; (b) removing the acidic agent; and (c) contacting the sample with a reducing agent. In some embodiments, the acidic agent has a pH of from 1.0 to 4.0. In some embodiments, the reducing agent has a pH from 8.0 to 10.0. In some embodiments, the step of contacting the sample with an acidic agent is performed prior to contacting the sample with a reducing agent. In other embodiments, the step of contacting the sample with a reducing agent is performed prior to contacting the sample with an acidic agent.

In some embodiments, the step of contacting the sample with the acidic agent of the method disclosed herein is performed for less than or equal to about 20 minutes, 15, 10, 5, 4, 3, 2, or 1 minutes. In some embodiments, the method disclosed herein comprises contacting the sample with the acidic agent for a period about 2 minutes to about 20 minutes, from about 2 minutes to about 15 minutes, from about 2 minutes to about 10 minutes, from about 2 minutes to about 5 minutes, from about 2 minutes to about 4 minutes, from about 2 minutes to about 3 minutes, or from about 1 minutes to about 2 minutes. In some embodiments, the step (a) of contacting with the acidic agent is performed for about 2 minutes.

In some embodiments, the step of contacting the sample with the reducing agent is performed for less than or equal to about 20 minutes, 15, 10, 9, 8, 7, 6, 5, 4, 3, or 2 minutes. In some embodiments, the step of contacting the sample with the reducing agent includes contacting the sample with the reducing agent for a period about 2 minutes to about 20 minutes, from about 2 minutes to about 15 minutes, from about 2 minutes to about 10 minutes, from about 2 minutes to about 8 minutes, from about 2 minutes to about 7 minutes, or from about 2 minutes to about 5 minutes. In some embodiments, the step of contacting the sample with the reducing agent is performed for about 5 minutes.

In some embodiments, the treatment with both acidic agent and reducing agent according to the methods disclosed herein are performed for less than or equal to about 40 minutes, for less than or equal to about 30 minutes, for less than or equal to about 25 minutes, for less than or equal to about 10 minutes, or for less than or equal to about 5 minutes. Existing methods of hematoxylin removal using strong acid may take at least an hour to up to a day or two and such a lengthy treatment of the sample with acid may degrade the sample, making it no longer suitable for subsequent analysis e.g., using fluorescence detection. According to the method of the present invention, the time for the de-staining process can be substantially reduced.

In some embodiments, during the step of contacting the sample with the acidic agent, the temperature of the sample is maintained at between about 0 °C and 10 °C, between about 20 °C and 30 °C, between about 35 °C and about 100 °C, between about 40 °C and about 90 °C, between about 45 °C and about 80 °C, or between about 50 °C and about 70 °C. In some embodiments, during the step of contacting the sample with the acidic agent, the temperature of the sample is maintained at between 20 °C and 30 °C. In some embodiments, the method disclosed herein further comprises applying heat to the sample so that, during the step of contacting the sample with the acidic agent, the sample and the acidic agent are maintained at a predetermined temperature while in contact. The predetermined temperature may be at between about 0 °C and 10 °C, between about 20 °C and 30 °C, between about 35 °C and about 100 °C, between about 40 °C and about 90 °C, between about 45 °C and about 80 °C, or between about 50 °C and about 70 °C.

In some embodiments, during the step of contacting the sample with the reducing agent, the temperature of the sample is maintained at between about 0 °C and 10 °C, between about 20 °C and 30 °C, between about 35 °C and about 100 °C, between about 40 °C and about 90 °C, between about 45 °C and about 80 °C, or between about 50 °C and about 70 °C. In some embodiments, during the step of contacting the sample with the reducing agent, the temperature of the sample is maintained at between 20 °C and 30 °C. In some embodiments, the method disclosed herein further comprises comprising applying heat to the sample so that, during the step of contacting the sample with the reducing agent, the sample and the reducing agent are maintained at a predetermined temperature while in contact. The predetermined temperature may be at between about 0 °C and 10 °C, between about 20 °C and 30 °C, between about 35 °C and about 100 °C, between about 40 °C and about 90 °C, between about 45 °C and about 80 °C, or between about 50 °C and about 70 °C.

In some embodiments, a heated slide platform is used for applying heat to the sample.

In some embodiments, the sample comprises cells. In some embodiments, the sample is a tissue-containing sample or a tissue section. In some embodiments, the sample is a formalin-fixed paraffin-embedded (FFPE) tissue section. In some embodiments, the sample has been deparaffinized and rehydrated prior to step (a). In some embodiments, the method further comprises, prior to step (a), deparaffinization and rehydration of the sample.

### A. De-staining

In some embodiments, the histological stain comprises hematoxylin.

As used herein, "H&E" means hematoxylin and eosin. H&E staining involves application of hematoxylin (the active form of the dye is called hematein, but it is commonly referred to as hematoxylin) and eosin. Without wishing to limit the present disclosure to any theory or mechanism, it is believed that during this process hematoxylin is oxidized to hematein and hematein subsequently binds to metal ions including aluminum (Al³⁺), iron (Fe³⁺) and chromium (Cr³⁺). This results in a blue color staining of nuclei of cells (and a few other cellular components, such as keratohyalin granules). Thus, the mechanism of staining of nuclei by hematoxylin is by binding of the dye-metal complex to arginine-rich basic nucleoproteins such as histones as well as deoxyribonucleic acid (DNA). The nuclear staining is followed by counterstaining with an aqueous or alcoholic solution of eosin, which colors other, eosinophilic structures in various shades of red, pink, and orange. The eosinophilic structures are generally composed of intracellular or extracellular protein. Other methods that involve using hematoxylin includes Masson's Trichrome used to stain connective tissues.

### B. Acidic Treatment

The method according to the present invention comprises treatment with an acidic agent. Without wishing to limit the present disclosure to any theory or mechanism, it is believed that acidic treatment is effective in disrupting the bond between the oxidized hematoxylin and mordant (e.g., Al³⁺, Fe³⁺, or Cr³⁺) that is bound to histones as well as deoxyribonucleic acid (DNA) in the sample.

According to the invention, the acidic agent has a pH from 1.0 to 4.0. In some embodiments, the acidic agent has a pH of 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, or 4.0.

In some embodiments, the acidic agent has a pH of from 1.0 to 2.0. In some embodiments, the acidic agent has a pH of from 1.0 to 1.9, from 1.0 to 1.8, from 1.0 to 1.7, from 1.0 to 1.6, from 1.0 to 1.5, from 1.0 to 1.4, from 1.0 to 1.3, from 1.0 to 1.2, or from 1.0 to 1.1. In some embodiments, the acidic agent has a pH of from 1.1 to 2.0, from 1.2 to 2.0, from 1.3 to 2.0, from 1.4 to 2.0, from 1.5 to 2.0, from 1.6 to 2.0, from 1.7 to 2.0, from 1.8 to 2.0, or from 1.9 to 2.0. In some embodiments, the acidic agent has a pH from 1.1 to 1.9, from 1.2 to 1.8, from 1.3 to 1.7, from 1.4 to 1.6.

In some embodiments, the acidic agent has a pH of from 2.0 to 4.0. In some embodiments, the acidic agent has a pH of from 2.0 to 3.6. In some embodiments, the acidic agent has a pH of from 2.0 to 3.5. In some embodiments, the acidic agent has a pH of from 2.0 to 3.4. In some embodiments, the acidic agent has a pH of from 2.0 to 3.3. In some embodiments, the acidic agent has a pH of from 2.0 to 3.2. In some embodiments, the acidic agent has a pH of from 2.0 to 3.1. In some embodiments the acidic agent has a pH of from 2.0 to 3.0. In some embodiments, the acidic agent has a pH of from 2.0 to 2.9. In some embodiments, the acidic agent has a pH of from 2.0 to 2.8. In some embodiments, the acidic agent has a pH of from 2.0 to 2.7. In some embodiments, the acidic agent has a pH of from 2.0 to 2.6. In some embodiments, the acidic agent has a pH of from 2.0 to 2.5.

In some embodiments, the acidic agent has a pH of from 2.5 to 3.3. In some embodiments, the acidic agent has a pH of from 2.5 to 3.6. In some embodiments, the acidic agent has a pH of from 2.5 to 3.5. In some embodiments, the acidic agent has a pH of from 2.5 to 3.4. In some embodiments, the acidic agent has a pH of from 2.5 to 3.3. In some embodiments, the acidic agent has a pH of from 2.5 to 3.2. In some embodiments, the acidic agent has a pH of from 2.5 to 3.1. In some embodiments, the acidic agent has a pH of from 2.5 to 3.0. In some embodiments, the acidic agent has a pH of from 2.5 to 2.9. In some embodiments, the acidic agent has a pH of from 2.5 to 2.8. In some embodiments, the acidic agent has a pH of from 2.5 to 2.7. In some embodiments, the acidic agent has a pH of from 2.5 to 2.6.

In some embodiments, the acidic agent has a pH of from 2.8 to 3.6. In some embodiments, the acidic agent has a pH of from 2.8 to 3.5. In some embodiments, the acidic agent has a pH of from 2.8 to 3.4. In some embodiments, the acidic agent has a pH of from 2.8 to 3.3. In some embodiments, the acidic agent has a pH of from 2.8 to 3.2. In some embodiments, the acidic agent has a pH of from 2.8 to 3.1. In some embodiments, the acidic agent has a pH of from 2.8 to 3.0. In some embodiments, the acidic agent has a pH of from 2.8 to 2.9.

In some embodiments, the acidic agent includes about 0.5 % to about 2% HCl (v/v), about 1% to about 2% HCl (v/v), or about 1% to about 1.5 % HCl (v/v). In another embodiment, the acidic agent includes about 70% ethanol (v/v).

In some embodiments, the molarity of acid is from 0.01 M to 0.5M. In some embodiments, the molarity of acid is from about 0.01 M to 0.4 M, from 0.05 M to 0.4 M, from 0.1 M to 0.4 M, from 0.2 M to 0.4 M or from 0.2 M to 0.3 M. In some embodiments, the molarity of acid is from about 0.01 M to 0.3 M, from 0.05 M to 0.3 M, from 0.1 M to 0.3 M, or from 0.2 M to 0.3 M. In some embodiments, the molarity of acid is from about 0.01 M to 0.2 M, from 0.05 M to 0.2 M, from 0.1 M to 0.2 M, or from 0.15 M to 0.2 M. In some embodiments, the molarity of acid is from about 0.01 M to 0.2 M, from 0.05 M to 0.2 M, from 0.1 M to 0.2 M, or from 0.15 M to 0.2 M. In some embodiments, the molarity of acid is about 0.01, 0.05, 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, or 0.50 M. For example, in some embodiments, the acid comprises 0.12 M hydrochloric acid.

In some embodiments, the acidic agent comprises an acidic agent selected from hydrochloric acid, sulfuric acid, nitric acid, hydrobromic acid, hydroiodic acid, sulfamic acid, perchloric acid, or a combination thereof. The acid may be any strong acid as understood and known in the art. A strong acid is any acid that is completely dissociated or ionized in an aqueous solution.

In some embodiments, the acidic agent comprises hydrochloric acid.

In some embodiments, the acidic agent comprises a solvent. In some embodiments, the solvent comprises water, ethylene glycol, polyethylene glycol, propylene glycol, ethanol, methanol, or a combination thereof. In some embodiments, the solvent in the acidic agents comprises water. In some embodiments, the solvent of the acidic agent comprises an alcohol (e.g., ethanol). In some embodiments, the solvent comprises ethylene glycol.

In some embodiments, the acidic agent comprises about 1% HCl in about 70% ethanol.

### C. Reducing agents

The method according to the present invention employs reduction reactions. In some embodiments, the method comprises contacting the sample with a reducing agent.

In some embodiments, the reducing agent has a pH of from 8.0 to 10.0. Note the pH of the reducing agent as used herein indicates the pH of the solution before applying it to the sample. In some embodiments, the reducing agent has a pH of from 8.0 to 9.5. In some embodiments, the reducing agent has a pH of from 8.0 to 9.2. In some embodiments, the reducing agent has a pH of from 8.0 to 9.0. In some embodiments, the reducing agent has a pH of from 8.0 to 8.9. In some embodiments, the reducing agent has a pH of from 8.0 to 8.8. In some embodiments, the reducing agent has a pH of from 8.0 to 8.7. In some embodiments, the reducing agent has a pH of from 8.0 to 8.6. In some embodiments, the reducing agent has a pH of from 8.0 to 8.5. In some embodiments, the reducing agent has a pH of from 8.0 to 8.4. In some embodiments, the reducing agent has a pH of from 8.0 to 8.3. In some embodiments, the reducing agent has a pH of from 8.0 to 8.2. In some embodiments, the reducing agent has a pH of from 8.0 to 8.1.

In some embodiments, the reducing agent has a pH of from 8.1 to 9.0. In some embodiments, the reducing agent has a pH of from 8.2 to 9.0. In some embodiments, the reducing agent has a pH of from 8.3 to 9.0. In some embodiments, the reducing agent has a pH of from 8.4 to 9.0. In some embodiments, the reducing agent has a pH of from 8.5 to 9.0. In some embodiments, the reducing agent has a pH of from 8.6 to 9.0. In some embodiments, the reducing agent has a pH of from 8.7 to 9.0. In some embodiments, the reducing agent has a pH of from 8.8 to 9.0. In some embodiments, the reducing agent has a pH of from 8.9 to 9.0.

In some embodiments, the reducing agent has a pH of from 8.1 to 8.9. In some embodiments, the reducing agent has a pH of from 8.2 to 8.8. In some embodiments, the reducing agent has a pH of from 8.3 to 8.7. In some embodiments, the reducing agent has a pH of from 8.4 to 8.6.

In some embodiments, the reducing agent has a pH of about 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0.

In some embodiments, the reducing agent comprises a reducing agent selected from sodium borohydride, sodium cyanoborohydride, potassium bromate, sodium sulfite, sodium dithionite, sodium thiosulfate, sodium bisulfite, sodium triethylborohydride, and sodium triacetoxyborohydride, or a combination thereof.

In some embodiments, the reducing agent comprises a solvent. In some embodiments, the solvent comprises water, an alcohol, ethylene glycol, propylene glycol, polyethylene glycol, or a combination thereof. In some embodiments, the solvent of the reducing agent comprises water. In some embodiments, the solvent of the reducing agent comprises an alcohol, such as one or more of ethanol, isopropanol, and/or methanol. In some embodiments, the solvent of the reducing agent comprises ethylene glycol.

In some embodiments, the reducing agent comprises about 1% sodium borohydride in ethanol, such as about 50% ethanol, about 60% ethanol, about 70% ethanol, about 80% ethanol, about 90% ethanol, and about 100% ethanol.

In some embodiments, the reducing agent comprises about 1% sodium borohydride in water. In some embodiments, the reducing agent comprises about 1% sodium borohydride in ethanol.

In some embodiments, the reducing agent comprises about 1% sodium borohydride in about 9.2 pH carbonate-bicarbonate buffer.

In some embodiments, the molarity of the reducing agent is from 0.1 M to 0.5 M. In some embodiments, the molarity of reducing agent is from 0.1 M to 0.5 M, from 0.2 M to 0.5 M, 0.3 M to 0.5 M, or from 0.4 M to 0.5 M. In some embodiments, the molarity of reducing agent is from 0.1 M to 0.4 M, from 0.2 M to 0.4 M, or from 0.3 M to 0.4 M. In some embodiments, the molarity of reducing agent is from 0.1 M to 0.3 M, from 0.15 M to 0.3 M, or from 0.2 M to 0.3 M. In some embodiments, the molarity of the reducing agent is about 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, or 0.50 M. For example, in some embodiments, the reducing agent comprises about 0.26 M sodium borohydride.

In some embodiments, the reducing agent comprises 0.05 M to 0.2 M sodium borohydride (e.g., a 0.13 M sodium borohydride). In some embodiments, the reducing agent comprises 0.1 M to 0.5 M sodium cyanoborohydride (e.g., 0.16 M sodium cyanoborohydride).

### D. Removal

In some embodiments, a removal step may be performed.

As used herein, the term "removing" (or "removal" or "removed") in reference to removing a histological stain from a sample means reducing the amount of histological stain in the sample to achieve a lowered signal over background result that permits a subsequent analysis of the sample using the same or a different analysis method. Accordingly, in some embodiments, while some residual histological stain may remain after the method disclosed herein is performed, the signal produced by any such residual histological stain may be reduced to a level that does not impair the subsequent analysis.

The term "removing" (or "removal" or "removed") when used in reference to removing an acidic agent or removing a reducing agent from a sample means to remove the activity of the acidic agent or the reducing agent in the sample, e.g., by physically depleting the agent from the sample; by applying a material that renders the agent non-functional (e.g., a neutralizing or degrading material that renders the agent non-functional), or by applying a condition that renders the agent non-functional (e.g., temperature, photoactivating light, etc. that renders the agent non-functional). As such, removing of an acidic agent may occur upon addition of a reducing agent.

In some embodiments, the acidic agent and/or the reducing agent is removed by mechanical means, e.g., by decanting, suction, positive pressure, and the like. In some embodiments, the acidic agent and/or reducing agent is removed by neutralization. In some embodiments, the acidic agent and/or reducing agent is removed by rinsing with a rinsing agent, which itself is subsequently optionally removed. A rinsing agent may comprise, e.g., water (which may be deionized water, a buffer, an alcohol mixture). In some embodiments, the acidic agent and/or reducing agent remains on the sample but its function is removed, e.g., by dilution, neutralization, decomposition, and the like.

If the method uses a rinsing agent after the acidic agent and a rinsing agent after the reducing agent, the two rinsing agents may be the same or they may be different.

### E. Embodiments comprising further analysis

As used herein, the term "histologically stained sample" means a biological sample that has been stained with a histological dye. The histologically stained sample may be an archived or stored sample (e.g., for a period of time, including minutes, hours, weeks, months, years, or even decades), or may be a sample prepared specifically for sequential staining using different analysis methods. The methods described herein may be used to remove a variety of histological stains. In an embodiment, the histological stain is H&E. In some embodiments, the methods disclosed herein may be employed in a process where H&E stain is been removed from a sample, and the sample is subjected to a subsequent analysis method. Additionally, the same detection channel used for imaging H&E stain becomes available for detection using another analysis method. In some embodiments, the methods disclosed herein may be applicable in detection of cellular features or molecular targets using a variety of analysis methods, e.g., immunohistochemistry, in situ hybridization, other protein and/or nucleic acid detection methods, or histological stains.

Examples of histological stains include hematoxylin and eosin (H&E) staining; Giesma staining, Gram staining, Periodic Acid Schiff Reaction staining, Papanicolaou staining, Masson's trichrome staining, Congo red staining, Musicarmine staining; silver staining. These can be performed using well-known methods (e.g., Thompson, Samuel W. Selected Histochemical and Histopathological Methods, Springfield, IL, 1966; Sheehan, D.C. and Hrapchak, B.B.: Theory and Practice of Histotechnology, 2nd Edition; Battelle Memorial Institute, Columbus, OH, 1987; Alturkistani HA, Tashkandi FM, Mohammedsaleh ZM. Histological Stains: A Literature Review and Case Study. Glob J Health Sci. 2015 Jun 25;8(3):72-9. Brown RC, Hopps HC. Staining of bacteria in tissue sections: a reliable gram stain method. Am J Clin Pathol. 1973 Aug;60(2):234-40; Dolan M. The role of the Giemsa stain in cytogenetics. Biotech Histochem. 2011 Apr;86(2):94-7; Al Drees A, Salah Khalil M, Soliman M. Histological and Immunohistochemical Basis of the Effect of Aminoguanidine on Renal Changes Associated with Hemorrhagic Shock in a Rat Model. Acta Histochem Cytochem. 2017 Feb 28;50(1):11-19.). Multiplex immunohistochemistry staining can be performed using a variety of methods, including the commercially available FlexVUE, FixVUE and U-VUE kits available from Ultivue, Inc, and other methods such as those described in "Overview of multiplex immunohistochemistry/immunofluorescence techniques in the era of cancer immunotherapy" (Cancer Commun (Lond). 2020 Apr; 40(4): 135-153). Detecting targets in the same sample may further provide spatial information about the targets in the sample. Methods disclosed herein may also be applicable in analytical applications where a limited amount of sample may be available for analysis and the same sample may have to be processed for multiple analyses. Thus, the methods disclosed herein may also facilitate multiple analyses of tissue sections.

The methods may further facilitate analyses based on detection methods that may be limited in the number of simultaneously detectable targets because of limitations of resolvable signals.

Accordingly, in some embodiments, a method of preparing a histologically stained sample for staining to detect at least one target is provided. In certain embodiments the method may include: (a) contacting the sample with an acidic agent; (b) contacting the sample with a reducing agent; and (c) staining the sample using a subsequent analysis method.

In some embodiments, the method further comprises, prior to step (a), detecting hematoxylin and eosin (H&E) staining.

In some embodiments, the subsequent analysis method step comprises contacting the sample with a staining agent to stain at least one target in the sample. In some embodiments, the method disclosed herein further comprises detecting the at least one target in the sample. In some embodiments, the sample may then undergo further sequential staining and detection of targets in multiple rounds of staining and signal removal. As such the method disclosed herein may be applicable to using another analysis method, such as IHC, in situ hybridization, or spatial transcriptomics methods, after a slide has been subjected to H&E staining.

In some embodiments, the subsequent analysis is a staining method.

In some embodiments, the subsequent analysis method is a histological staining method. In some embodiments, the subsequent analysis method is an immunohistochemical staining method. In some embodiments, the subsequent analysis method is a nucleic acid detection method. In some embodiment, the subsequent analysis method is a protein detection method. In some embodiments, the subsequent analysis method is a spatial transcriptomics method. Thus, the subsequent analysis method can target any molecule, including, for example, one or more nucleic acids, proteins, lipids, carbohydrates, glycans, glycoproteins, oligonucleotides, or a combination thereof.

Protein detection methods may include, e.g., imaging mass spectrometry (MIBI), imaging mass cytometry (IMC), multiplexed or single-plex immunohistochemistry, and other protein analysis method performed in solution or *in situ.*

Spatial transcriptomics methods may include, for example, multiplexed single-molecule in situ hybridization methods (e.g., MERFISH, seqFISH, osmFISH, Nanostring CosMx: Chen et al., Spatially Resolved, Highly Multiplexed RNA Profiling in Single Cells. Science 2015, 348 (6233), aaa6090.; Shah et al., In Situ Transcription Profiling of Single Cells Reveals Spatial Organization of Cells in the Mouse Hippocampus. Neuron 2016, 92 (2), 342-357; Lubeck et al., Single-Cell in Situ RNA Profiling by Sequential Hybridization. Nat Methods 2014, 11 (4), 360-361.; Codeluppi et al., Spatial Organization of the Somatosensory Cortex Revealed by OsmFISH. Nat Methods 2018, 15 (11), 932-935; He et al., High-Plex Multiomic Analysis in FFPE at Subcellular Level by Spatial Molecular Imaging. bioRxiv January 2, 2022, p 2021.11.03.467020), multiplex in situ sequencing (e.g., STARMAP, ISS: Wang et al., Three-Dimensional Intact-Tissue Sequencing of Single-Cell Transcriptional States. Science 2018, 361 (6400), eaat5691; Hilscher et al., In Situ Sequencing: A High-Throughput, Multi-Targeted Gene Expression Profiling Technique for Cell Typing in Tissue Sections. In In Situ Hybridization Protocols; Nielsen, B. S., Jones, J., Eds.; Methods in Molecular Biology; Springer US: New York, NY, 2020; pp 313-329), and/or in situ RNA capture methods (e.g., ST/Visium, SLIDE-seq, HDST: Ståhl et al., Tissue Sections by Spatial Transcriptomics. Science 2016, 353 (6294), 78-82.; Rodriques et al., Slide-Seq: A Scalable Technology for Measuring Genome-Wide Expression at High Spatial Resolution. Science 2019, 363 (6434), 1463-1467; Vickovic et al., High-Definition Spatial Transcriptomics for in Situ Tissue Profiling. Nat Methods 2019, 16 (10), 987-990).

In an embodiment, the subsequent analysis method is multiplexed fluorescence IHC, which is performed prior to a further analysis method involving in situ RNA capture.

In some embodiments, the method disclosed herein further comprises:
(1) contacting the sample being tested for the presence of at least one target with at least one corresponding target-specific binding partners, wherein each target-specific binding partner of different specificity is linked to a different nucleic acid strand;
(2) contacting the sample with labeled imager strands having complementarity to a nucleic acid strand linked to the target-specific binding partner;
(3) imaging the sample to detect labeled bound imager strands
(4) optionally removing signal from the labeled bound imager strands; and
(5) optionally repeating at least some of steps (2)-(4) at least once with a labeled imager strand having a unique composition relative to at least one other labeled imager strand,
thereby detecting the location of the at least one target in the sample.

In some embodiments, any detectable label may be used to label imager strands, and, in some embodiments, the moiety is optically detectable.

In an embodiment, a fluorescent label is used. General categories of fluorescent labels include organic dyes, biological fluorophores, quantum dots, and nanoparticles including carbon dots. Specific fluorescent dyes include fluorescein, rhodamine, cyanine dyes, ALEXA dyes, DYLIGHT dyes, and ATTO dyes. The Examples herein describe use of four spectrally distinct fluorescent labels in a single round of detection. It is possible to use more than four spectrally overlapping fluorophores in one round of detection. Use of software to assist in detecting fluorophores having overlapping signals is known (see for example, US6,750,964). A variety of fluorescent dyes and filters are commercially available, allowing the methods described herein to be performed using any feasible number of fluorescent labels. As described herein, in an embodiment, the methods can be performed using a single fluorescent label; two fluorescent labels; three fluorescent labels; four fluorescent labels; five fluorescent labels; six fluorescent labels; seven fluorescent labels; eight fluorescent labels; and greater than eight fluorescent labels. Generally, when using more than one fluorescent label, signals are detected in different detection channels which correspond to different regions of the light spectrum. The table below shows four detection channels and representative fluorophores.

**Table 1. Detection channels and exemplary fluorophores**

| **Microscope Detection Channel** | **Emission Detection Wavelength Range (nm)** | **Example Fluorophores** |
|---|---|---|
| "FITC" | 510 - 530 | FITC, FAM, Fluorescein, Cy2, Alexa Fluor 488, Atto 488, |
| "TRITC" | 570 - 590 | TRITC, TAMRA, Cy3, Quasar 570, Alexa Fluor 568, Atto 550 |
| "Cy5" | 670 - 690 | Cy5, Alexa Fluor 647, Atto 647N, Quasar 670 |
| "Cy7" | 750 - 780 | Cy7, Alexa Fluor 750, Atto 740, IRDye 750 |

Further methods for multiplex imaging for detecting multiple targets in a same sample are described in US Patent No. 10,294,510, titled "High-throughput and highly multiplexed imaging with programmable nucleic acid probes," and US Patent Application No. 2018/0164308, titled "Methods for multiplex imaging using labeled nucleic acid imaging agents," US Patent Application No. 2019/ 0376956, titled "Multiplexed catalyzed reporter deposition," WO/2021/007099 titled "Improved Multiplexing Method", VVO/2020/123961, titled "Methods and Compositions For Sequentially Detecting Targets".

In some embodiments, the methods disclosed herein may result in multiple fluorescent images and corresponding bright-field morphological images obtained using the H&E staining. In some embodiments, a control stain (e.g., a DAPI nuclear stain) may be used to overlay the nucleus stained with H&E in the bright-field images with the fluorescent images.

In the imaging steps, sometimes referred to as detecting steps, a signal from a histological stain, IHC stain or in situ hybridization, may be detected using a detection system. The nature of the detection system used may depend upon the nature of the signal generators used. The detection system may include a charge coupled device (CCD) detection system, a fluorescent detection system, an electrical detection system, a photographic film detection system, a chemiluminescent detection system, an enzyme detection system, an optical detection system, a near field detection system, or a total internal reflection (TIR) detection system.

### F. Samples

As used herein, the term "sample" means any natural or man-made biological fluid, cell, tissue, or fraction thereof, or other material, that includes or is suspected to include a target. A sample can be derived from a prokaryote or eukaryote and therefore can include cells from, for example, animals, plants, or fungi. Accordingly, a sample includes a specimen obtained from one or more individuals or can be derived from such a specimen. In some embodiments, the sample comprises a cell. In some embodiments, the sample comprises a tissue.

In some embodiments, the sample is a tissue sample. As used herein, "tissue sample" means a collection of cells obtained from a tissue of an individual. The tissue may contain nucleated cells with chromosomal material. The source of the tissue sample may be solid tissue, as from a fresh, frozen, FFPE, and/or preserved organ or tissue sample, or biopsy, or aspirate, or blood or any blood constituents, or bodily fluids, such as cerebral spinal fluid, amniotic fluid, peritoneal fluid, or interstitial fluid, or cells from any time in gestation or development of the subject. The tissue sample may also be primary or cultured cells or cell lines, or culture tissues. The tissue sample may contain compounds which are not naturally intermixed with the tissue in nature, such as preservatives, anticoagulants, buffers, fixatives, nutrients, antibiotics, or the like.

In some embodiments, the tissue sample comprises a tissue section. As used herein, "section" of a tissue sample means a single part or piece of a tissue sample, for example, a thin slice of tissue or cells cut from a tissue sample. It is understood that multiple sections of tissue samples may be subjected to analysis according to the present invention. In some embodiments, the selected portion or section of tissue comprises a population of cells. In some embodiments, the selected portion or section of tissue comprises a homogeneous population of cells. In some embodiments, the selected portion or section of tissue comprises a heterogeneous population of cells. In some embodiments, the selected portion comprises a region of tissue, e.g., an organ, a tumor, the stroma, the lumen as non-limiting examples. The selected portion or section can be as small as one cell or two cells, or could represent many thousands of cells, for example.

Any tissue sample from one or more individuals may be used. In an embodiment, the sample is a tissue from an individual. In an embodiment, the sample is a tissue microarray sample comprising tissue from one or more individuals. Examples of tissue samples that may be used include, but are not limited to, breast, prostate, ovary, colon, lung, endometrium, stomach, salivary gland, or pancreas. The tissue sample can be obtained by a variety of procedures including, but not limited to, surgical excision, aspiration, or biopsy.

The tissue may be fresh or frozen. In some embodiments, the tissue sample is a tissue section of brain, adrenal glands, colon, small intestines, stomach, heart, liver, skin, kidney, lung, pancreas, testis, ovary, prostate, uterus, thyroid, and spleen of a mammal (e.g., human or mouse). The methods of the present disclosure may be applied to any type of tissue, including, for example, cancer tissue (including from any cancer).

A sample used in a method described herein may be an unfixed or fixed biological sample. In some embodiments, the sample is fixed. Any fixative may be used. In an embodiment, the fixative is a solution containing an aldehyde. In some embodiments, the sample is fixed in a solution containing formalin. In some embodiments, the sample is paraffin embedded. In some embodiments, the sample is a formalin-fixed paraffin-embedded (FFPE) tissue sample.

A tissue sample may be disposed on a surface, such as a slide, flow cell, 3D matrix, or particle.

### II. Kits

In some embodiments, disclosed is a kit for removing a histological stain from a sample. The kit includes (a) an acidic agent having pH from 1.0 to 4.0 and (b) a reducing agent having a pH from 8.0 to 10.0 disclosed herein. The kit may further include a rinsing agent disclosed herein (e.g., deionized water). The kit may further include a staining reagent disclosed herein (e.g., an immunohistochemistry reagent, or an in situ fluorescent hybridization reagent). In some embodiments, the agents (a) and (b) and optional (c) and (d) are separately contained in respective containers in the kit. The kit may include instructions for use.

In some embodiments, one or more of the aforementioned methods may be automated and may be performed using automated systems. In some embodiments, all the steps may be performed using automated systems.

### III. Use

The methods disclosed herein may be used in analytic, diagnostic, and therapeutic applications. In some embodiments, the methods disclosed herein may be used in analysis by imaging, e.g., immunohistochemistry, including chromogenic and immunofluorescence detection, or nucleic acid hybridization methods using, e.g., fluorescence detection, or other methods. Analysis of samples from an individual, according to the methods described herein, may be used diagnostically (e.g., to identify individuals who have a particular disease, have been exposed to a particular toxin or are responding well to a particular therapeutic or organ transplant) and prognostically (e.g., to identify individuals who are likely to develop a particular disease, respond well to a particular therapeutic or be accepting of a particular organ transplant). The methods disclosed herein, may facilitate accurate and reliable analysis of a plurality of targets (e.g., disease markers) from the same sample.

### EXAMPLES

### Example 1.

This example describes comparison of hematoxylin stain removal according to the present disclosure, comparing treatment with both an acidic agent and reducing agent, with treatment with only acidic agent or only reducing agent. Comparison with positive control (no H&E staining) or the negative control (H&E staining without removal) is also shown.

**Preparation of tissue slides:** human formalin-fixed, paraffin-embedded (FFPE) tonsil tissue sections disposed on slides (Amsbio LLC, Cambridge, MA) were first baked for 30 min at 60 °C . For H&E staining, slides were processed on an Epredia Gemini automated slide staining device to stain with hematoxylin and eosin. Slides that were previously stained with H&E may also be used in this procedure.

**Acidic agent:** An acid bath containing 1% HCl in 70% EtOH was prepared by mixing 6 mL of 10 N HCl to 500 mL of 70% denatured ethanol.

**Reducing agent:** 1% NaBH₄ solution in water adjusted to pH 9 with 1M NaOH.

Similar results are observed using 1% NaBH₄ solution in 70% denatured ethanol (about 63% ethanol; 3-4% isopropanol and methanol) or 100% denatured ethanol (about 90% ethanol; 5% methanol, 5% isopropanol).

Eight (8) tissue slides were treated according to the schemes listed in the table below.

| | |
|---|---|
| Positive control (no H&E) | Slide #01 |
| Negative control (H&E with no removal) | Slide #02 |
| no H&E + Reducing agent only | Slide #03 |
| H&E + Reducing agent only | Slide #04 |
| no H&E + Acidic agent + Reducing agent | Slide #05 |
| H&E + Acidic agent + Reducing agent | Slide #06 |
| no H&E + Acidic agent only | Slide #07 |
| H&E + Acidic agent only | Slide #08 |

For all applicable slides, the acidic agent contacted the tissue for 2 minutes. For all applicable slides, the reducing agent contacted the tissue for 10 minutes.

Antigen retrieval was performed by incubating the slides in epitope retrieval solution 2 (AR9640, Leica Biosystems) for 20 minutes at 100 °C. The slides were then washed and subjected to immunostaining for four different targets (Ki67, PD1, CD3, CD68) using FixVUE^{™} panels (Ultivue, Cambridge, MA) according to the manufacturer's manual. Note that eosin was effectively removed during the antigen retrieval step and does not impair subsequent immunostaining.

Figures 1A-1C show results of analysis of positive control (where no H&E staining was performed; Slide #01) and negative control (where H&E staining was performed but no hematoxylin removal was performed; Slide #02). Fig. 1C shows brightfield image of the negative control. As shown in Fig. 1B, significantly less fluorescent signal was observed if no hematoxylin removal was performed. As shown in Fig. 1C, pattern of remaining hematoxylin strongly resembles region of reduced fluorescence shown in Fig. 1B.

Figures 2A-2D show fluorescent signals of the tissue where no H&E staining was performed and no treatment (Fig. 2A; Slide #01), the reduction treatment (Fig. 2B; Slide #03), the acidic treatment (Fig. 2D; Slide #07), or both treatments (Fig. 2C; Slide #05) were performed. Figures 3A-3D show fluorescent signals of the tissue where H&E staining was performed but no treatment (Fig. 3A; Slide #02), the reduction treatment (Fig. 3B; Slide #04), the acidic treatment (Fig. 3D; Slide #08), or both treatments (Fig. 3C; Slide #06) were performed.

Figures 4A-8B show comparison of fluorescence signals from previously H&E-stained slides for which H&E was removed, followed by immunostaining for Ki67, PD1, CD3, and CD68 (slide #06), along with the positive controls (with no H&E staining; Slide #01). As shown in the figures, the quality of immunostaining in each detection channel (Ki67, PD1, CD3, and CD68, respectively) was comparable to the positive control without prior H&E staining (Figs. 5A, 6A, 7A, and 8A) when the hematoxylin was removed according to the present disclosure (see Figs. 5B, 6B, 7B, and 8B).

### Example 2.

This example describes comparison of hematoxylin stain removal according to the present disclosure, under different reducing agent conditions.

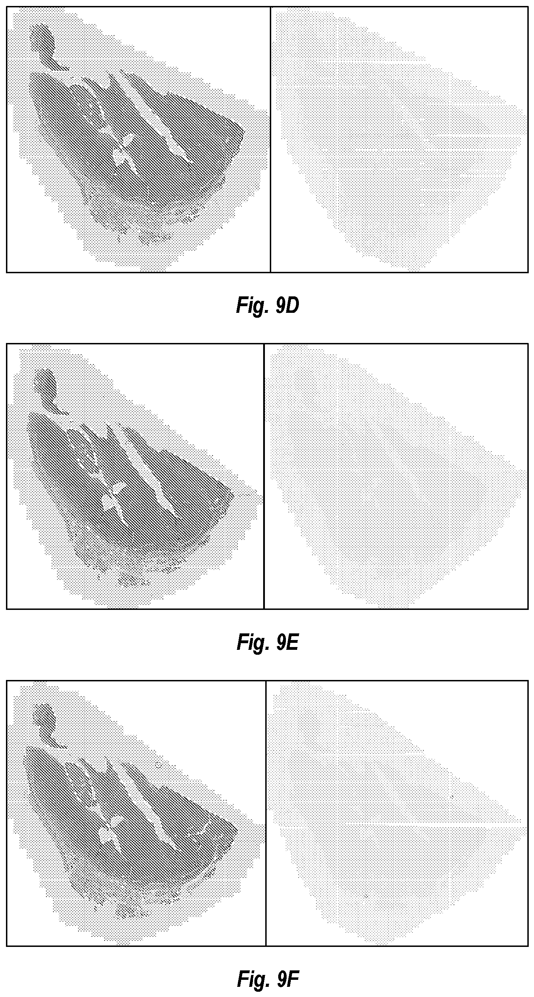

Human formalin-fixed, paraffin-embedded (FFPE) tonsil tissue sections stained by standard H&E protocol were de-coverslipped by soaking in xylenes for two days. Slides were then rehydrated through 1 minute incubation through a series of graded alcohols (100%, 95%, 90%, 80%, water). The slides were then incubated in a solution of 1% (v/v) HCl in 70% ethanol for 2 minutes, followed by:
1. 10 min in 1% NaBH₄ in 90% ethanol; 5% methanol, 5% isopropanol (Fig. 9A)
2. 5 min in 1% NaBH₄ in 90% ethanol; 5% methanol, 5% isopropanol (Fig. 9B)
3. 10 min in 1% NaBH₄ in 63% ethanol, 3% isopropanol; 3% methanol (Fig. 9C)
4. 5 min in 1% NaBH₄ in 63% ethanol, 3% isopropanol; 3% methanol (Fig. 9D)
5. 10 min in 1% NaBH₄ in pH 9.2 carbonate-bicarbonate buffer (Fig. 9E)
6. 5 min in 1% NaBH₄ in pH 9.2 carbonate-bicarbonate buffer (Fig. 9F)

After this differential treatment, slides were coverslipped and brightfield microscopic images were taken. Figs. 9A-9F show that hematoxylin was removed according to the present disclosure.

## Claims

1. A method of removing a histological stain from a histologically stained sample, comprising:
(a) contacting the sample with an acidic agent, wherein the acidic agent has a pH from 1.0 to 4.0; and
(b) contacting the sample with a reducing agent, wherein the reducing agent has a pH from 8.0 to 10.0,
thereby removing the histological stain from the histologically stained sample.

2. The method of claim 1, wherein the acidic agent is removed from the sample.

3. The method of claim 1, wherein the reducing agent is removed from the sample.

4. The method of any one of claims 1-3, wherein the acidic agent and/or reducing agent is removed from the sample using a rinsing agent.

5. The method of any one of claims 1-4, wherein the histological stain comprises hematoxylin and optional further comprises eosin.

6. The method of any one of claims 1-5, wherein the acidic agent comprises hydrochloric acid (HCl), sulfuric acid, nitric acid, hydrobromic acid, hydroiodic acid, sulfamic acid, perchloric acid, or a combination thereof and wherein the acidic agent further comprises a solvent selected from water, ethylene glycol, polyethylene glycol, propylene glycol, ethanol, methanol, or a combination thereof.

7. The method of claim 6, wherein the acidic agent comprises 1% HCl in 70% ethanol.

8. The method of any one of claims 1-7, wherein the reducing agent comprises a reducing agent selected from sodium borohydride, sodium cyanoborohydride, sodium sulfite, sodium dithionite, sodium thiosulfate, sodium bisulfite, sodium triethylborohydride, and sodium triacetoxyborohydride, or a combination thereof and wherein the reducing agent comprises a solvent selected from water, an alcohol, ethanol, ethylene glycol, and propylene glycol, or a combination thereof.

9. The method of claim 8, wherein the reducing agent comprises 1% sodium borohydride in water.

10. The method of any one of claims 1-9, wherein the step of contacting the sample with the acidic agent is performed for less than or equal to 20 minutes, 15, 10, 5, 4, 3, 2, or 1 minutes; and/or wherein the step of contacting the sample with the reducing agent is performed for less than or equal to 20 minutes, 15, 10, 9, 8, 7, 6, 5, 4, 3, or 2 minutes.

11. The method of any one of claims 1-10, wherein during the step of contacting the sample with the acidic agent, the temperature of the sample is maintained at between 20 °C and 30 °C; and/or wherein during the step of contacting the sample with the reducing agent, the temperature of the sample is maintained at between 20 °C and 30 °C.

12. The method of any one of claims 1-11, further comprising applying heat to the sample so that, during the step of contacting the sample with the acidic agent, the sample and the acidic agent are maintained at a predetermined temperature while in contact; and/or further comprising applying heat to the sample so that, during the step of contacting the sample with the reducing agent, the sample and the reducing agent are maintained at a predetermined temperature while in contact.

13. The method of any one of claims 1-12, further comprising:
(1) contacting the tissue sample being tested for the presence of at least one target with at least one corresponding target-specific binding partner, wherein each target-specific binding partner of different specificity is linked to a different nucleic acid strand;
(2) contacting the tissue sample with labeled imager strands having complementarity to a nucleic acid strand linked to the target-specific binding partner; and
(3) imaging the sample to detect labeled bound imager strands;
(4) optionally repeating at least some of steps (2)-(3) at least once with a labeled imager strand having a unique composition relative to at least one other labeled imager strand,
thereby detecting the location of the at least one target in the sample.

14. The method of any one of claims 1-13, wherein the sample comprises cells or tissue.

15. A kit for removing a histological stain from a tissue sample, comprising
(a) an acidic agent having pH from 1.0 to 4.0;
(b) a reducing agent having a pH from 8.0 to 10.0;
(c) optionally a rinsing agent; and
(d) optionally a staining reagent.

## Patentansprüche

1. Verfahren zum Entfernen einer histologischen Färbung von einer histologisch gefärbten Probe, das Folgendes umfasst:
(a) das In-Kontakt-Bringen der Probe mit einem sauren Mittel, wobei das saure Mittel einen pH-Wert von 1,0 bis 4,0 aufweist, und
(b) das In-Kontakt-Bringen der Probe mit einem Reduktionsmittel, wobei das Reduktionsmittel einen pH-Wert von 8,0 bis 10,0 aufweist,
wodurch die histologische Färbung von der histologisch gefärbten Probe entfernt wird.

2. Verfahren nach Anspruch 1, wobei das saure Mittel aus der Probe entfernt wird.

3. Verfahren nach Anspruch 1, wobei das Reduktionsmittel aus der Probe entfernt wird.

4. Verfahren nach einem der Ansprüche 1 bis 3, wobei das saure Mittel und/oder das Reduktionsmittel unter Verwendung eines Spülmittels aus der Probe entfernt werden.

5. Verfahren nach einem der Ansprüche 1 bis 4, wobei die histologische Färbung Hämatoxylin umfasst und gegebenenfalls weiters Eosin umfasst.

6. Verfahren nach einem der Ansprüche 1 bis 5, wobei das saure Mittel Salzsäure (HCl), Schwefelsäure, Salpetersäure, Bromwasserstoffsäure, Iodwasserstoffsäure, Amidosulfonsäure, Perchlorsäure oder eine Kombination davon umfasst und wobei das saure Mittel weiters ein Lösungsmittel umfasst, das aus Wasser, Ethylenglykol, Polyethylenglykol, Propylenglykol, Ethanol, Methanol und einer Kombination davon ausgewählt ist.

7. Verfahren nach Anspruch 6, wobei das saure Mittel 1 % HCl in 70 % Ethanol umfasst.

8. Verfahren nach einem der Ansprüche 1 bis 7, wobei das Reduktionsmittel ein Reduktionsmittel umfasst, das aus Natriumborhydrid, Natriumcyanoborhydrid, Natriumsulfit, Natriumdithionit, Natriumthiosulfat, Natriumbisulfit, Natriumtriethylborhydrid und Natriumtriacetoxyborhydrid und einer Kombination davon ausgewählt ist, und wobei das Reduktionsmittel ein Lösungsmittel umfasst, das aus Wasser, einem Alkohol, Ethanol, Ethylenglykol und Propylenglykol und einer Kombination davon ausgewählt ist.

9. Verfahren nach Anspruch 8, wobei das Reduktionsmittel 1 % Natriumborhydrid in Wasser umfasst.

10. Verfahren nach einem der Ansprüche 1 bis 9, wobei der Schritt des In-Kontakt-Bringens der Probe mit dem sauren Mittel 20 min, 15, 10, 5, 4, 3, 2 oder 1 min oder weniger lang durchgeführt wird;
und/oder wobei der Schritt des In-Kontakt-Bringens der Probe mit dem Reduktionsmittel 20 min, 15, 10, 9, 8, 7, 6, 5, 4, 3 oder 2 min oder weniger lang durchgeführt wird.

11. Verfahren nach einem der Ansprüche 1 bis 10, wobei während des Schritts des In-Kontakt-Bringens der Probe mit dem sauren Mittel die Temperatur der Probe auf zwischen 20 °C und 30 °C gehalten wird; und/oder wobei während des Schritts des In-Kontakt-Bringens der Probe mit dem Reduktionsmittel die Temperatur der Probe auf zwischen 20 °C und 30 °C gehalten wird.

12. Verfahren nach einem der Ansprüche 1 bis 11, das weiters die Zufuhr von Wärme zur Probe umfasst, so dass während des Schritts des In-Kontakt-Bringens der Probe mit dem sauren Mittel, die Probe und das saure Mittel auf einer vorbestimmten Temperatur gehalten werden, während sie in Kontakt stehen; und/oder wobei das Verfahren weiters die Zufuhr von Wärme zur Probe umfasst, so dass während des Schritts des In-Kontakt-Bringens der Probe mit dem Reduktionsmittel, die Probe und das Reduktionsmittel auf einer vorbestimmten Temperatur gehalten werden, während sie in Kontakt stehen.

13. Verfahren nach einem der Ansprüche 1 bis 12, das weiters Folgendes umfasst:
(1) das In-Kontakt-Bringen der Gewebeprobe, die auf die Gegenwart zumindest eines Targets getestet wurde, mit zumindest einem entsprechenden targetspezifischen Bindungspartner, wobei jeder targetspezifische Bindungspartner mit unterschiedlicher Spezifität an einen unterschiedlichen Nukleinsäure-Strang gebunden wird;
(2) das In-Kontakt-Bringen der Gewebeprobe mit markierten Abbildungs-Strängen, die eine Komplementarität zu einem Nukleinsäure-Strang aufweisen, der an den targetspezifischen Bindungspartner gebunden ist; und
(3) das Abbilden der Probe, um markierte gebundene Abbildungs-Stränge zu detektieren;
(4) gegebenenfalls das zumindest einmalige Wiederholen zumindest mancher der Schritte (2) bis (3) mit einem markierten Abbildungs-Strang, der in Bezug auf zumindest einen anderen markierten Abbildungs-Strang eine einzigartige Zusammensetzung aufweist, wodurch die Position des zumindest einen Targets in der Probe detektiert wird.

14. Verfahren nach einem der Ansprüche 1 bis 13, wobei die Probe Zellen oder Gewebe umfasst.

15. Set zum Entfernen einer histologischen Färbung von einer Gewebeprobe, das Folgendes umfasst:
(a) ein saures Mittel, das einen pH-Wert von 1,0 bis 4,0 aufweist;
(b) ein Reduktionsmittel, das einen pH-Wert von 8,0 bis 10,0 aufweist;
(c) gegebenenfalls ein Spülmittel; und
(d) gegebenenfalls ein Färbungsreagens.

## Revendications

1. Procédé d'élimination d'une coloration histologique d'un échantillon coloré histologiquement, comprenant les étapes consistant à :
(a) mettre en contact l'échantillon avec un agent acide, dans lequel l'agent acide présente un pH compris entre 1,0 et 4,0 ; et
(b) mettre en contact l'échantillon avec un agent réducteur, dans lequel l'agent réducteur présente un pH de 8,0 à 10,0, en éliminant ainsi la coloration histologique de l'échantillon coloré histologiquement.

2. Procédé selon la revendication 1, dans lequel l'agent acide est éliminé de l'échantillon.

3. Procédé selon la revendication 1, dans lequel l'agent réducteur est éliminé de l'échantillon.

4. Procédé selon l'une quelconque des revendications 1 à 3, dans lequel l'agent acide et/ou l'agent réducteur est éliminé de l'échantillon en utilisant un agent de rinçage.

5. Procédé selon l'une quelconque des revendications 1 à 4, dans lequel la coloration histologique comprend de l'hématoxyline et comprend en outre facultativement de l'éosine.

6. Procédé selon l'une quelconque des revendications 1 à 5, dans lequel l'agent acide comprend de l'acide chlorhydrique (HCI), de l'acide sulfurique, de l'acide nitrique, de l'acide bromhydrique, de l'acide iodhydrique, de l'acide sulfamique, de l'acide perchlorique ou une combinaison de ceux-ci et dans lequel l'agent acide comprend en outre un solvant choisi parmi l'eau, l'éthylène glycol, le polyéthylène glycol, le propylène glycol, l'éthanol, le méthanol ou une combinaison de ceux-ci.

7. Procédé selon la revendication 6, dans lequel l'agent acide comprend 1 % d'HCI dans de l'éthanol à 70 %.

8. Procédé selon l'une quelconque des revendications 1 à 7, dans lequel l'agent réducteur comprend un agent réducteur choisi parmi le borohydrure de sodium, le cyanoborohydrure de sodium, le sulfite de sodium, le dithionite de sodium, le thiosulfate de sodium, le bisulfite de sodium, le triéthylborohydrure de sodium et le triacétoxyborohydrure de sodium, ou une combinaison de ceux-ci, et dans lequel l'agent réducteur comprend un solvant choisi parmi l'eau, un alcool, l'éthanol, l'éthylène glycol et le propylène glycol, ou une combinaison de ceux-ci.

9. Procédé selon la revendication 8, dans lequel l'agent réducteur comprend 1 % de borohydrure de sodium dans de l'eau.

10. Procédé selon l'une quelconque des revendications 1 à 9, dans lequel l'étape consistant à mettre en contact l'échantillon avec l'agent acide est effectuée pendant ou pendant moins de 20 minutes, 15, 10, 5, 4, 3, 2 ou 1 minute ; et/ou dans lequel l'étape consistant à mettre en contact l'échantillon avec l'agent réducteur est effectuée pendant ou pendant moins de 20 minutes, 15, 10, 9, 8, 7, 6, 5, 4, 3 ou 2 minutes.

11. Procédé selon l'une quelconque des revendications 1 à 10, dans lequel pendant l'étape de mise en contact de l'échantillon avec l'agent acide, la température de l'échantillon est maintenue entre 20° et 30°C ; et/ou dans lequel pendant l'étape de mise en contact de l'échantillon avec l'agent réducteur, la température de l'échantillon est maintenue entre 20°C et 30°C.

12. Procédé selon l'une quelconque des revendications 1 à 11, comprenant en outre l'application de chaleur à l'échantillon de telle sorte que pendant l'étape de mise en contact de l'échantillon avec l'agent acide, l'échantillon et l'agent acide sont maintenus à une température prédéterminée pendant qu'ils sont en contact ; et/ou comprenant en outre l'application de chaleur à l'échantillon de telle sorte que pendant l'étape de mise en contact de l'échantillon avec l'agent réducteur, l'échantillon et l'agent réducteur sont maintenus à une température prédéterminée pendant qu'ils sont en contact.

13. Procédé selon l'une quelconque des revendications 1 à 12, comprenant en outre l'étape consistant à :
(1) mettre en contact l'échantillon de tissu testé pour la présence d'au moins une cible avec au moins un partenaire de liaison spécifique à une cible correspondant, dans lequel chaque partenaire de liaison spécifique à une cible de spécificité différente est lié à un brin d'acide nucléique différent ;
(2) mettre en contact l'échantillon de tissu avec des brins d'imageur marqués présentant une complémentarité avec un brin d'acide nucléique lié au partenaire de liaison spécifique à une cible ; et
(3) imager l'échantillon pour détecter des brins d'imageur liés marqués ;
(4) répéter facultativement au moins certaines des étapes (2) à (3) au moins une fois avec un brin d'imageur marqué présentant une composition unique par rapport à au moins un autre brin d'imageur marqué, en détectant ainsi l'emplacement de la au moins une cible dans l'échantillon.

14. Procédé selon l'une quelconque des revendications 1 à 13, dans lequel l'échantillon comprend des cellules ou un tissu.

15. Kit pour enlever une coloration histologique à partir d'un échantillon de tissu, comprenant
(a) un agent acide présentant un pH de 1,0 à 4,0 ;
(b) un agent réducteur présentant un pH de 8,0 à 10,0 ;
(c) facultativement un agent de rinçage ; et
(d) facultativement un réactif de coloration.
